# EUROPEAN PATENT APPLICATION

(11) **EP 3 988 126 A1**
(43) Date of publication of application: **27.04.2022**
(21) Application number: 20202995.5
(22) Date of filing: 21.10.2020
(51) Int. Cl.: A61L 2/07, A47C 23/00, B66C 1/00, B66F 9/00

(54) **DEVICE FOR DISINFECTING MATTRESSES**

(71) Applicant: Weber Hospital Systems B.V., 1689 AP Zwaag (NL)
(72) Inventor: Geelhoed, Cornelis Marinus, 3846 JK Harderwijk (NL); Sierkstra, Erik, 1625 MD Hoorn (NL)
(74) Representative: 't Jong, Bastiaan Jacob

(57) **Abstract**

The invention relates to a device for disinfecting mattresses, which device comprises:
- an entry portal defining a feed opening;
- a plurality of steam nozzles arranged on the entry portal and distributed around the circumference of the feed opening;
- a conveyor, such as a roller conveyor or conveyor belt, which conveyor has a feed end and a discharge end, and which conveyor extends through the entry portal; and
- a steam supply connected to the steam nozzles for providing the steam nozzles with steam.

## Description

The invention relates to a device for disinfecting mattresses. In particular in hospitals it is desired or even required to disinfect hospital beds including the mattresses.

Hospital bed mattresses are typically provided with a special ticking which can easily be cleaned with a cleaning fluid. Such special ticking is typically provided with a polyurethane coating, such that a cleaning fluid is not immediately absorbed by the ticking. In order to allow for some ventilation, when a patient is in the hospital bed, the ticking is provided with a perforation.

Such mattresses can be cleaned by hand with a sponge or towel. Care should be taken not to use too much cleaning fluid, as the fluid will penetrate the mattress via the perforation and then it will take a long time before the mattress is dry again.

Another method of cleaning or disinfecting a mattress is by using ultraviolet light. The UV-light damages the bacteria and other micro-organisms, but does not remove these destroyed bacteria and micro-organisms from the mattress.

It is further known to automate the cleaning or disinfecting of hospital beds. To this end, a hospital bed is positioned onto a conveyor belt, where the hospital bed is first scanned to determine the type of bed and whether the bed is in a correct position, typically with head and foot part in horizontal position. Then the mattress is removed from the bed frame for separate cleaning. The bed frame is transported further, such that an articulated robot can spray the bed with a disinfecting fluid, while the mattress is cleaned with a disinfecting fluid in a separate path. Finally, the mattress is positioned back on the bed frame.

Using a disinfecting fluid in such an automated process requires that a sufficient amount of fluid is used and has sufficient time to act to ensure that the bed and the mattress are indeed disinfected. Then the bed and the mattress need to be rinsed with hot water and finally dried. This takes a substantial amount of time per mattress and per bed. If the mattress is still wet, it cannot be positioned on the bed frame and this will delay the automated process of cleaning and disinfecting hospital beds. And if cleaning fluid has penetrated the ticking of the mattress through the perforation, it will even take longer before the bed can be made with bed sheets.

It is an object of the invention to reduce or even remove the above mentioned disadvantages.

This object is achieved by the invention with a device for disinfecting mattresses, which device comprises:
- an entry portal defining a feed opening;
- a plurality of steam nozzles arranged on the entry portal and distributed around the circumference of the feed opening;
- a conveyor, such as a roller conveyor or conveyor belt, which conveyor has a feed end and a discharge end, and which conveyor extends through the entry portal; and
- a steam supply connected to the steam nozzles for providing the steam nozzles with steam.

Steam is typically a mixture of water in gas phase and a mist of water droplets. Dry steam has a low amount of water droplets, while wet steam has a high amount of water droplets. Within the scope of this invention, also hot air can be used, which can be considered a very dry steam as air typically contains some amount of water.

With the device according to the invention a mattress can be transported over the conveyor and through the entry portal. The steam nozzles on the entry portal will eject steam onto the mattress from all directions, such that the surface of the mattress is disinfected.

The advantage of using steam is that it is environmental friendly, bacteria will not get resistant and the high temperature of the steam in combination with the little amount of water in the steam ensures that any condensation is quickly evaporated, such that the mattress will leave the device virtually dry. Furthermore, if steam penetrates the perforation of the ticking of the mattress, it will quickly evaporate and due to the limited amount of steam not cause the mattress to get moist on the inside.

In an embodiment of the device according to the invention the entry portal comprises an elongate bottom part, two upstanding side parts arranged on either side of the elongate bottom part, and an elongate top part arranged between the side parts and at a distance from the elongate bottom part, wherein the plurality of steam nozzles are mounted on the elongate bottom part, the side parts and the elongate top part.

The bottom part, side parts and top part will typically provide a rectangular feed opening, which corresponds to the shape of a regular mattress.

Preferably, the side parts are movable relative to each other in a direction parallel to elongate bottom part. This allows for adjusting the width of the feed opening to correspond with the width of the mattress, such that the steam nozzles can be positioned close to the surface of the mattress and optimize the disinfecting action of the steam.

It is furthermore preferred if the elongate top part is movable towards and away from the elongate bottom part. This allows for adjusting the height of the feed opening and to adjust to the thickness of the mattress conveyed through the device.

In a very preferred embodiment of the device according to the invention the elongate top part is rotatable around an axis parallel to the longitudinal axis of the elongate top part, such that the steam nozzles can be directed between a position perpendicular to the support surface of the conveyor and a position parallel to the support surface of the conveyor belt.

As the top part can be moved up and down, it can also be brought in front of the mattress and behind the mattress. By rotating the steam nozzles in a position parallel to the support surface, the leading side and trailing side of the mattress can thus also be subjected to the steam by moving the top part up and down.

Yet another embodiment of the device according to the invention further comprises an exit portal defining an exit opening, a plurality of air knives arranged on the exit portal and distributed around the circumference of the exit opening and an air supply connected to the air knives for providing the air knives with air.

The air knives will remove any condensation on the mattress to ensure that the mattress will exit the device virtually dry and can instantly be positioned back on a disinfected bed frame. Preferably, the air knives are also movable, such that the distance between the air knives and the mattress can be optimized.

Yet another embodiment of the device according to the invention further comprises two lift devices arranged adjacent to the feed end of the conveyor and adjacent the discharge end of the conveyor respectively, wherein each lift device comprises a vacuum gripper movable in gravitational direction between a first position and a second position for lifting a mattress between a hospital bed and the feed end and the discharge end respectively of the conveyor.

The lift devices can lift the mattress from a bed and lift the mattress to a height where the entry portal and conveyor of the device according to the invention are positioned. The mattress is then fed through the entry portal such that the mattress is disinfected by steam from the steam nozzles and finally picked up by the second lift device and lowered back onto the bed frame.

As mattresses of hospital beds typically have a coating of for example polyurethane, a vacuum gripper can easily grip the mattresses by vacuum.

These and other features of the invention will be elucidated in conjunction with the accompanying drawings.
Figure 1 shows a perspective view of an installation for automatic disinfecting of hospital beds.
Figure 2 shows in perspective view with some parts hidden the device for disinfecting mattresses according to the invention.
Figure 3 shows a bottom view of the device according to the invention as shown in figure 2.
Figure 4 shows a perspective view of the device according to the invention and the second lift device.
Figure 5A and 5B show a side view of the device according to the invention in two positions.

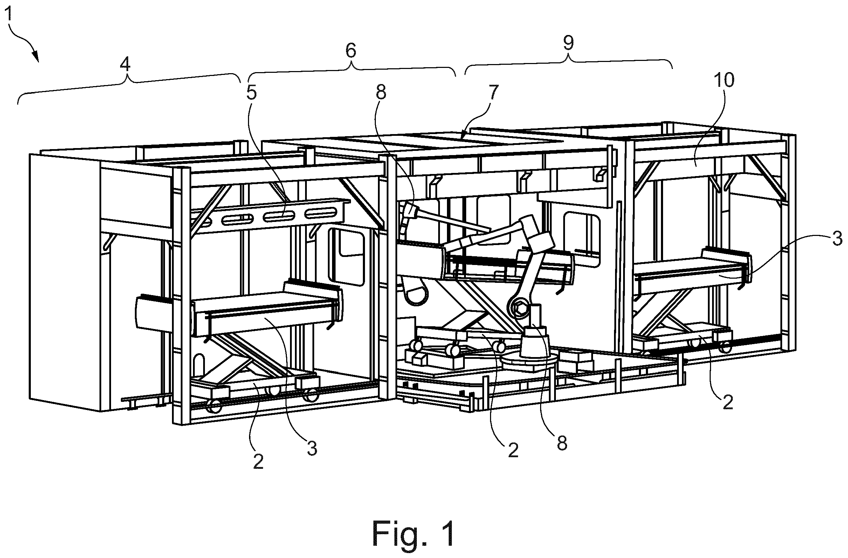

In figure 1 an installation 1 is shown for disinfecting hospital beds 2, 3. The installation 1 has a first zone 4 in which hospital beds 2, 3 are measured to determine which type the bed is and if the bed is in the right position. Then the mattress 3 is lifted off the bed frame 2 by a first lift device 5.

The bed frame 2 is transported to a second zone 6, while the mattress 3 is transported overhead into a device 7 for disinfecting the mattress 3.

In the second zone 6 two articulate robots 8 are positioned, which will clean the bed frame 2 with steam by moving an elongate steam head along the bed frame 2 in a programmed way and depending on the type of bed as detected in the first zone 4.

The mattress 3 is meanwhile disinfected by the device 7 and transported further into the third zone 9. The bed frame 2 is also transported into this third zone 9, such that second lifting means 10 can lower the mattress 3 back onto the bed frame 2.

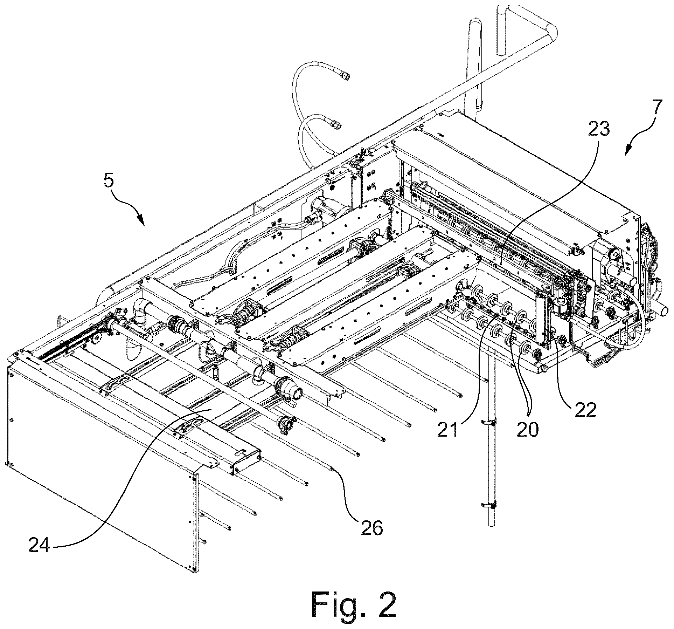

Figure 2 shows the device 7 for disinfecting mattresses in more detail. The device 7 has a roller conveyor 20, which extends through an entry portal formed by a bottom part 21, side parts 22 and a top part 23, which are all provided with steam nozzles.

Furthermore, a first lift device 5 is positioned in front of the entry portal 21, 22, 23. The lift device 5 has a vacuum gripper 24, which can be moved up and down to lift a mattress 3 from a bed frame 2 (see figure 1). After the mattress 3 is lifted in front of the entry portal 21, 22, 23, a grating 25 closes the lifting device 5, such that the mattress cannot fall out and can be transported over the roller conveyor 20 through the entry portal 21, 22, 23.

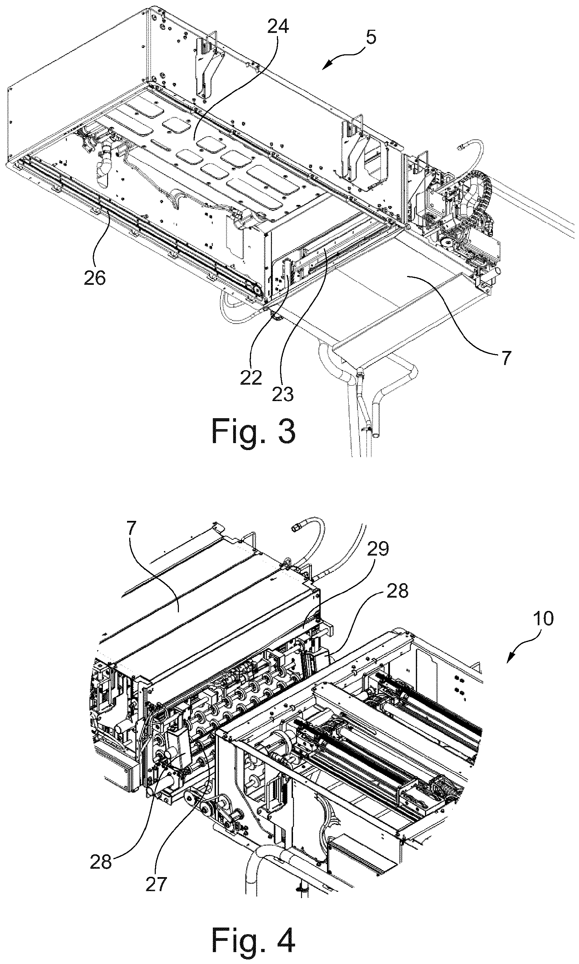

Figure 3 shows a bottom view of the first lift device 5. The first lift device 5 is shown in open position, wherein the grating 25 is moved out of view along a guide 26 and the vacuum gripper 24 is ready to be lowered in order to grip a mattress.

Figure 4 shows a perspective view of the device 7 according to the invention, which shows the exit portal composed out of air knives 27, 28, 29, which will blow a passing mattress dry. A second lift device 10 will pick up the dried and cleaned mattress and position the mattress back on a bed frame 2.

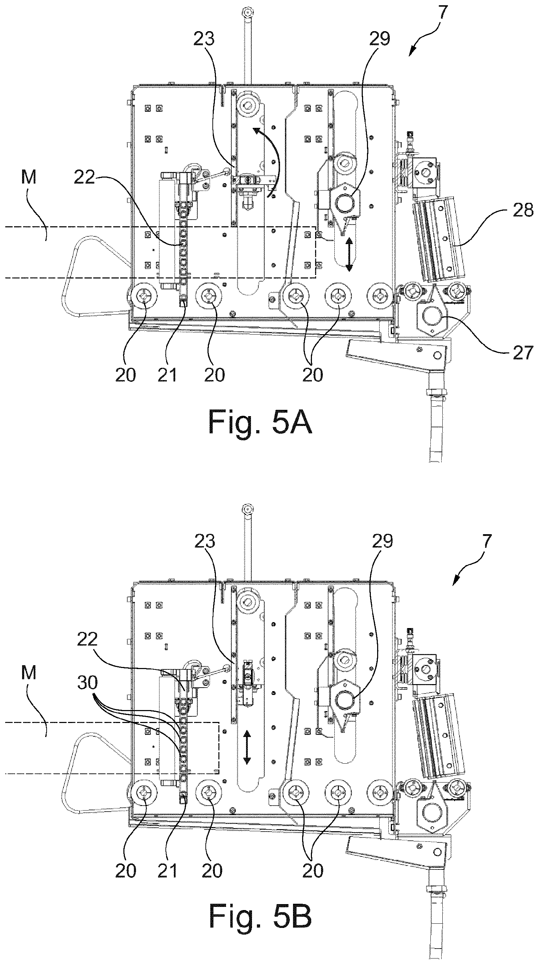

Figure 5A and 5B show the device 7 in side view in two different positions. A mattress M is shown in dashed line in front of the entry portal 21, 22, 23. From this side view, it is clear that the entry portal has a plurality of steam nozzles 30. When the mattress M is transported by the conveyor 20 into the device, the nozzles 30 will disinfect the mattress M.

The upper part 23 is rotatable along the longitudinal axis, such that the nozzles can be directed in a horizontal position (as shown in figure 5A) and in a vertical position (as shown in figure 5B). In the horizontal position the upper part 23 can be moved up and down such that the front side, or leading side, of the mattress M can also be thoroughly cleaned as well as the trailing side.

After the front side of the mattress M is disinfected, the nozzles of the upper part 23 are positioned in a vertical direction (as shown in figure 5B) for cleaning the top surface of the mattress M. The mattress M is then conveyed further and along the air knives 27, 28, 29 to dry the mattress M.

The upper air knife 29 and preferably also the side knives 28 are movable to adjust to the size of the mattress M.

## Claims

1. Device for disinfecting mattresses, which device comprises:
- an entry portal defining a feed opening;
- a plurality of steam nozzles arranged on the entry portal and distributed around the circumference of the feed opening;
- a conveyor, such as a roller conveyor or conveyor belt, which conveyor has a feed end and a discharge end, and which conveyor extends through the entry portal; and
- a steam supply connected to the steam nozzles for providing the steam nozzles with steam.

2. Device according to claim 1, wherein the entry portal comprises an elongate bottom part, two upstanding side parts arranged on either side of the elongate bottom part, and an elongate top part arranged between the side parts and at a distance from the elongate bottom part, wherein the plurality of steam nozzles are mounted on the elongate bottom part, the side parts and the elongate top part.

3. Device according to claim 2, wherein the side parts are movable relative to each other in a direction parallel to elongate bottom part

4. Device according to claim 2 or 3, wherein the elongate top part is movable towards and away from the elongate bottom part.

5. Device according to claim 4, wherein the elongate top part is rotatable around an axis parallel to the longitudinal axis of the elongate top part, such that the steam nozzles can be directed between a position perpendicular to the support surface of the conveyor and a position parallel to the support surface of the conveyor belt.

6. Device according to any of the preceding claims, further comprising an exit portal defining an exit opening, a plurality of air knives arranged on the exit portal and distributed around the circumference of the exit opening and an air supply connected to the air knives for providing the air knives with air.

7. Device according to any of the preceding claims, further comprising two lift devices arranged adjacent to the feed end of the conveyor and adjacent the discharge end of the conveyor respectively, wherein each lift device comprises a vacuum gripper movable in gravitational direction between a first position and a second position for lifting a mattress between a hospital bed and the feed end and the discharge end respectively of the conveyor.
